# FASCICULE DE BREVET EUROPEEN

(11) **EP 2 825 302 B1**
(45) Date de publication et mention de la délivrance du brevet: **24.04.2019**
(21) Numéro de dépôt: 13715904.2
(22) Date de dépôt: 14.03.2013
(51) Int. Cl.: B01J 19/00, G01N 33/543, C40B 50/18, C40B 60/14

(54) **DISPOSITIF DE GREFFAGE MICRO-STRUCTURE DE PROTEINES SUR UN SUBSTRAT**
VORRICHTUNG ZUR MIKROSTRUKTURIERTEN PFROPFUNG VON PROTEINEN AUF EIN SUBSTRAT
DEVICE FOR THE MICROSTRUCTURED GRAFTING OF PROTEINS ONTO A SUBSTRATE

(30) Priorité: 14.03.2012 FR 1252304
(43) Date de publication de la demande: 21.01.2015
(73) Titulaire: Alveole, 75005 Paris (FR); Centre National de la Recherche Scientifique (C.N.R.S.), 75794 Paris Cedex 16 (FR); Université Bordeaux Segalen, 33076 Bordeaux Cedex (FR)
(72) Inventeur: STUDER, Vincent, 33000 Bordeaux (FR); AZIOUNE, Ammar, 33700 Merignac (FR)
(74) Mandataire: Osha Liang
(86) Numéro de dépôt international: PCT/EP2013/055294
(87) Numéro de publication internationale: WO 2013/135844

(56) Documents cités:
- EP-A1- 1 517 178
- WO-A1-99/42813
- WO-A1-2006/084482
- WO-A2-99/41007
- WO-A2-02/072791
- FINK JENNY ET AL: "Comparative study and improvement of current cell micro-patterning techniques", LAB ON A CHIP, ROYAL SOCIETY OF CHEMISTRY, vol. 7, no. 6, 1 juin 2007 (2007-06-01), pages 672-680, XP002509909, ISSN: 1473-0197, DOI: 10.1039/B618545B

## Description

### Domaine technique de l'invention

L'invention concerne le domaine général du greffage de protéines sur un substrat selon des motifs et des concentrations différentes ou impression de protéines ou impression, l'impression étant obtenue par un moyen de greffage photochimique. L'invention concerne en particulier le domaine du greffage photochimique d'une pluralité, c'est-à-dire d'au moins deux protéines sur un substrat, de façon automatisée ou automatisable.

Une protéine est, au sens de la présente demande, une macromolécule biologique composée d'un grand nombre de chaînes d'acides aminés liés entre eux par des liaisons peptidiques. Une protéine rassemble un nombre d'acides aminés grand, par opposition aux peptides ou oligopeptides qui en contiennent un faible nombre. Les protéines assurent les fonctions des cellules vivantes.

La disposition sur un substrat de couches imprimées ou surfaciques de protéines dans des concentrations variables et des configurations ou motifs différents, est essentielle dans l'étude « in vitro », hors d'un organisme vivant, des cellules vivantes car elle permet de recréer un environnement de protéines aussi complexe que souhaité, rendant de moins en moins nécessaires les essais « in vivo », notamment sur des animaux.

Il est ainsi souhaitable dans l'art antérieur de disposer de moyens d'impression de protéines qui soient versatiles ou aptes à recréer, sur un substrat, tout motif ou patron ou « pattern », à partir de solutions de protéines ou d'ADN ou de molécules biologiques, de concentration données, par exemple contenues dans des réservoirs.

Il est aussi souhaitable de pouvoir imprimer industriellement une pluralité de protéines aussi simplement qu'une seule.

### Exposé de l'art antérieur

Des exemples de procédé de greffage de molécules sur un substrat sont décrits dans les documents de brevet WO 2006/084482 A1, WO 99/41007 A2, EP 1517178 A1 et WO 02/072791 A2.

Les solutions envisageables dans l'art antérieur pour l'impression photochimique d'une ou plusieurs protéines sur un même substrat souffrent de limitations importantes qui en empêchent l'application industrielle.

L'impression photochimique consiste à greffer ou fixer chimiquement sur une surface des molécules, sélectivement dans des zones illuminées de la surface, par adhésion photo-induite.

Un moyen photochimique d'impression ou de greffage est ainsi constitué dans l'art antérieur, d'une façon générale, d'un substrat, d'un moyen optique d'illumination du substrat, d'un moyen fluidique de faire parvenir à la surface du substrat un fluide contenant, en solution aqueuse une molécule biologique comme une protéine, un oligopeptide ou de l'ADN à greffer sur le substrat, et d'une molécule adhésive en présence de l'illumination ou colle moléculaire photo-adhésive, molécule qui peut être soit déposée en couche sur le substrat, soit présente en solution dans le fluide avec la protéine.

Lorsque la molécule adhésive est en couche solide déposée sur le substrat, le moyen de greffage photochimique est qualifié de « greffage en couche » dans la présente demande.

Lorsque la molécule adhésive est en solution dans un liquide au contact du substrat, le moyen de greffage photochimique est qualifié de « greffage en solution » dans la présente demande.

Concernant le greffage en couche, qui est une première technique de greffage de l'art antérieur, il utilise un substrat traité avec une couche solide, solidaire du substrat, photo-adhésive pour de « petites » molécules, à imprimer, et recouvrant entièrement la surface du substrat entre ce substrat et une solution des petites molécules. Les petites molécules en cause sont notamment des nucléotides afin d'obtenir une synthèse d'ADN ou des peptides pour obtenir une synthèse d'oligopeptides. Le greffage en couche est limité à des molécules « petites » devant la taille d'une protéine, au sens ou ces molécules ne doivent pas posséder une propriété de greffage « non-spécifique» sur le substrat, à l'échelle de temps de l'utilisation souhaitée du motif imprimé sur le substrat. La propriété de greffage « non-spécifique » est observée lorsque la taille d'une molécule photo-imprimée croît et cette propriété se caractérise notamment par un envahissement du substrat par la molécule en dehors des motifs photo-imprimés, dans un intervalle de temps rendant le substrat inutilisable en pratique, par écrantage du motif imprimé.

Un moyen d'illumination courant pour le greffage en couche consiste, de façon connue, en une matrice de micro-miroirs commandée digitalement et apte à réaliser un motif quelconque par basculement de chacun des micro-miroirs qui la composent, la matrice servant d'objet à un objectif qui forme une image micro-structurée du motif quelconque, sur la surface de la couche photo-adhésive en contact avec la solution contenant les petites molécules.

L'impression de multiples petites molécules est possible avec un moyen de greffage en couche, bien qu'elle nécessite des couches de protection pour les molécules déjà déposées, avant chaque nouvelle impression, ce qui complique industriellement l'utilisation de cette première technique.

Toutefois, les protéines présentant justement la propriété de greffage non-spécifique, les moyens de greffage en couche se révèlent inadaptés, dans l'art antérieur, à l'impression d'une seule protéine et a fortiori à l'impression d'une pluralité de protéines.

Concernant le greffage en solution qui est une seconde technique de greffage de l'art antérieur, il utilise un substrat traité par une couche antiadhésive pour une protéine et une colle photo-adhésive à la fois pour la couche photo-adhésive et pour la protéine, la colle photo-adhésive étant présente dans une solution aqueuse contenant la protéine, solution qui est mise au contact du substrat et illuminée. La présence d'un moyen dédié à prévenir le greffage non-spécifique sur le substrat, sous la forme de la couche antiadhésive pour les protéines, rend cette seconde technique adaptée à l'impression d'une seule protéine.

Toutefois, le greffage en solution utilise un masque de photolithographie sans aucune optique de focalisation et requière ainsi un film de solution de la protéine et de la colle photo-adhésive, qui soit le plus mince possible entre le masque et la couche antiadhésive, typiquement quelques microns. Il est utilisé ainsi une goutte de solution écrasée entre un masque et un substrat plan, positionnés de façon fixe l'un par rapport à l'autre. Cette structure opto-mécanique rend problématique la généralisation de cette seconde technique à l'impression d'une pluralité de protéines pour au moins deux raisons.

Premièrement, il est difficile d'envisager d'automatiser le changement de protéine dans le film par des moyens micro-fluidiques de pompage, changeant la solution. En effet, la perte de charge augmente avec la diminution d'épaisseur du film ou pellicule de fluide et cette épaisseur doit être minimale pour maximiser la qualité optique, une épaisseur typique est de 5 microns et une précision de 1 micron sur cette valeur étant considérée souhaitable. Les variations de planéité relatives du substrat et du masque étant inévitables, il se révèle que le dimensionnement d'un dispositif micro-fluidique adapté à un changement de solution, donc de protéine, automatique est difficilement envisageable pour cette seconde technique.

Deuxièmement, il est impératif d'utiliser autant de masques que de protéines à imprimer. Ce qui suppose un moyen d'alignement ou de positionnement des masques sur le substrat, non seulement dans le plan de ce substrat mais aussi vis-à-vis du parallélisme entre le substrat et le masque, celui-ci influant optiquement sur le motif imprimé. Il en résulte la nécessité de positionner en trois dimensions chaque masque par rapport au substrat. De plus, pour tenir compte des variations de planéité relative du masque et du substrat, il est a priori nécessaire d'utiliser des masques et un substrat dont les défauts de planéité sont très faibles ou de recalculer chaque masque pour chaque substrat, ce qui est inenvisageable industriellement.

En conséquence, pour ces deux raisons, la seconde technique ne peut être utilisée aisément dans l'art antérieur pour l'impression industrielle de plus d'une protéine sur un substrat.

L'impression de plusieurs protéines en séquence ou l'une après l'autre, sur un substrat, de façon automatisable et industrielle est donc un problème difficile pour l'art antérieur et la conception d'un système rapide d'impression de plusieurs protéines, sur un même substrat, avec une qualité compatible avec la restitution de motifs micro-structurés ou présentant des détails d'une finesse de l'ordre du micromètre ou micron, ne semble pas envisageable dans l'art antérieur à l'invention.

### Exposé de l'invention

Dans ce contexte, l'invention est un dispositif pour le greffage micro-structuré de plusieurs protéines sur un substrat, tel que défini dans la revendication 1 annexée. Un tel dispositif comprend un substrat, une couche, une matrice, une source d'une lumière, un système optique, un premier réservoir destiné à recevoir une première solution aqueuse, un deuxième réservoir destiné à recevoir une seconde solution aqueuse et un circuit micro-fluidique, dans lequel la couche est disposée sur le substrat, la source est adaptée à éclairer la matrice par la lumière , la matrice est adaptée à propager la lumière selon un premier motif structuré, la matrice comprend des moyens optiques de remplacement du premier motif structuré par un second motif structuré, le système optique est adapté à former, sur la couche, une première image micro-structurée du premier motif, le circuit est adapté à contenir la première solution aqueuse, le circuit comprend une ouverture pour mettre en contact la première solution avec la couche au niveau de l'ouverture, le circuit comprend des moyens micro-fluidiques de remplacement de la première solution par la seconde solution, la couche comprend un polyéthylène glycol à sa surface.

Ladite matrice est une matrice de micro-miroirs propageant ladite lumière par réflexion.

Ledit système optique est un objectif de microscope.

Dans des variantes du dispositif ci-dessus :
- ladite source est un laser émettant à une longueur d'onde ultraviolette,
- ladite longueur d'onde ultraviolette vaut 365 nm.

L'invention concerne aussi un procédé de greffage micro-structuré de protéines sur un substrat utilisant le dispositif ci-dessus et comprenant les étapes suivantes :
- remplir le premier réservoir avec une première solution aqueuse comprenant une benzophénone et une première protéine,
- remplir ledit circuit micro-fluidique avec ladite première solution aqueuse pour mettre en contact la première solution et ladite couche, au niveau de ladite ouverture,
- former, à l'aide de ladite lumière, ladite première image micro-structurée dudit premier motif structuré, sur la couche, pour photo-imprimer ladite première protéine sur la couche.

Dans une variante du procédé ci-dessus :
- ladite première protéine est fluorescente.

L'invention concerne aussi le procédé ci-dessus comprenant les étapes suivantes :
- remplir le deuxième réservoir avec une deuxième solution aqueuse comprenant la benzophénone et une deuxième protéine,
- remplacer ladite première solution aqueuse par ladite seconde solution aqueuse pour mettre en contact la seconde solution et ladite couche, au niveau de ladite ouverture,
- remplacer ledit premier motif structuré par ledit second motif structuré pour photo-imprimer ladite seconde protéine sur la couche.

Dans une variante du procédé ci-dessus :
- ladite seconde protéine est fluorescente.

### Liste des figures

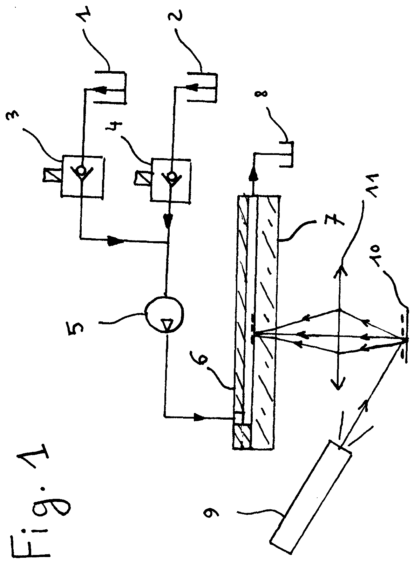

L'invention est décrite en référence à la figure 1 pour les numéros entre parenthèses.

La figure 1 représente un premier mode de l'invention dans le cas d'un système apte à imprimer deux protéines différentes sur un même substrat à l'aide de motifs définis par une matrice de micro-miroirs. Un laser (9) émettant dans l'ultraviolet est à cette fin disposé pour éclairer une matrice de micro-miroirs (10). Un objectif (11) tenant lieu de système optique, image la matrice (10), sur un substrat (7) transparent, par l'intérieur de ce substrat, sur une première surface du substrat. A l'extérieur du substrat, au contact de cette première surface est disposé un micro-canal (6) faisant partie d'un circuit fluidique comprenant en série avec une première entrée du micro-canal, une micro-pompe (5), un premier réservoir (1) pouvant alimenter la micro-pompe lorsqu'une première micro-vanne (3) commandée électriquement est ouverte par un calculateur non représenté, un second réservoir (2) pouvant alimenter la micro-pompe lorsqu'une seconde micro-vanne (4) commandée électriquement est ouverte par le calculateur, le micro-canal du circuit comprend aussi une sortie qui débouche sur un réservoir (8) de fuite ou de drainage.

### Description détaillée d'au moins un mode de réalisation de l'invention

Dans un premier mode de l'invention, une première protéine est contenue dans un premier réservoir (1) et une seconde protéine est contenue dans un second réservoir (2).

La première protéine est le Fibrinogène vert connu sous le nom de Fibrinogen-Alexa Fluor 488 et la seconde protéine est le Fibrinogène rouge connu sous le nom de Fibrinogen Alexa Fluor 546. Il est aussi possible de choisir la Fibronectine pour l'une des deux protéines ou pour les deux, en remplacement desdits fibrinogènes. Les protéines suivantes : Fibrinogène, Fibronectine, Laminine, Collagène et Vitronectine peuvent aussi être utilisées avec l'invention. Des protéines fluorescentes comme notamment le fibrinogène vert et le fibrinogène rouge sont avantageuses pour l'invention, à des fins de visualisation des impressions réalisées sur le substrat.

La première protéine est diluée dans un premier fluide ou première solution aqueuse ou tamponnée, contenu dans le premier réservoir, la première solution comprend aussi de l'eau et un premier moyen de greffage qui est une Benzophénone ou « benzoylbenzyltrimethylammonium chloride » pour son nom anglais ou «chlorure de benzoylbenzyltrimethylammonium» en français, dans une version soluble dans l'eau, de ce produit.

La seconde protéine est diluée dans un second fluide ou une seconde solution aqueuse ou tamponnée, contenu dans le second réservoir, la seconde solution comprend aussi de l'eau et un second moyen de greffage qui est aussi ladite Benzophénone.

Dans tout mode de l'invention, une solution utilisant un liquide autre que l'eau mais ne dénaturant pas les protéines, peut être utilisé.

Le premier réservoir est connecté à une première micro-vanne (3) et le second réservoir est connecté à une seconde micro-vanne (4). La première micro-vanne et la seconde micro-vanne sont connectées à une micro-pompe (5). La micro-pompe est connectée à une première extrémité d'un micro-canal (6) surmontant un substrat (7) en verre, qui est traité sur une première surface de ce substrat, par une fine couche, de l'ordre de 2nm, constituée de polyéthylène glycol ou PEG. Le substrat forme un couvercle pour le micro-canal qui présente une ouverture au niveau du substrat, de façon qu'un fluide, traversant le micro-canal, soit en contact avec la première surface du substrat ou plus précisément avec la fine couche de PEG ou encore avec le substrat traité.

Le micro-canal est ouvert à une seconde extrémité et laisse échapper un liquide le traversant dans un réservoir de fuite (8). Le cas échéant, si aucune pollution des réservoirs n'est à craindre, un système de recyclage du fluide s'échappant du micro-canal dans le réservoir d'où le fluide est issu, pourrait être prévu.

Une micro-pompe adaptée à l'invention sera par exemple une micro-pompe à débit constant non réversible.

Une micro-vanne adaptée à l'invention pourra être notamment un clapet anti-retour normalement bloqué par le fluide et qui est ouvert par une commande électrique. Tout autre tiroir hydraulique permettant la commutation ou le multiplexage des réservoirs dans une seule conduite reliée à la micro-pompe sera adapté à l'invention.

Dans toute la présente demande, le préfixe « micro » pour des termes du domaine technique de la fluidique n'est pas, limitatif en termes de dimension à des objets de la taille d'un micron mais signifie que les éléments fluidiques adoptés pour constituer le circuit ou système fluidique de l'invention, sont dimensionnés de façon aussi petite que possible pour éviter de gaspiller la première solution et la seconde solution lorsqu'elles sont mises en contact avec le substrat par les éléments fluidiques dudit circuit, puis évacuées dans le réservoir de fuite. Si une surface d'impression ou surface d'impression du substrat est fixée par exemple à un carré de 1cm par 1 cm, une profondeur du micro-canal ou extension du micro-canal à partir de la première surface, en dehors de cette première surface, pourra être ainsi de 200 microns, pour obtenir un volume de 20 microlitres de solution au-dessus de la surface d'impression. Le renouvellement pourra ainsi être limité à des quantités faibles de solution et de protéine, grâce à l'aspect micro-fluidique de certains moyens de l'invention, ce qui constitue un avantage industriel.

Dans toute la présente demande, les mots « impression sur le substrat » sont considérés synonymes de « impression sur la couche » lorsque la couche est un traitement de surface du substrat d'épaisseur faible devant celle du substrat. Ce qui est le cas de la couche et du substrat dans l'invention.

Le substrat est dans la présente demande du verre ou de l'ITO ou tout autre matériau transparent s'il doit être traversé par la lumière pour obtenir l'impression. De même, la couche est transparente si elle doit être traversée par la lumière pour obtenir l'impression. Dans le cas où un substrat serait naturellement antiadhésif pour les protéines, la couche serait alors entendue comme une couche superficielle du substrat sur laquelle a lieu le greffage, sans sortir de l'enseignement de l'invention.

Le dimensionnement d'un circuit fluidique ou micro-fluidique, compte-tenu des indications précédentes, qui est adapté à l'invention, peut être effectué, par un homme du métier de la micro-fluidique, sans difficulté particulière avec ses connaissances usuelles. L'invention comprendra ainsi un circuit micro-fluidique disposé entre la couche et le premier et le second réservoir de solution aqueuse contenant la première et la seconde protéine respectivement. Dans une généralisation de l'invention au niveau de son circuit fluidique, le circuit micro-fluidique comprendra des moyens de mettre en contact une sortie du circuit avec une pluralité de solutions aqueuses contenant chacune une protéine particulière et la benzophénone, en fonction par exemple d'une commande automatique par un calculateur selon un choix programmé. Dans le mode présenté, l'architecture fluidique correspondant à un multiplexeur à deux entrées, une par réservoir, et une sortie, au niveau du micro-canal, il est possible d'ajouter des réservoirs et des entrées au multiplexeur par des moyens fluidiques connus pour augmenter le nombre de solutions de protéines qui peuvent remplir le micro-canal. L'invention est ainsi présentée dans ce premier mode comme illustration de sa structure à deux protéines, sans être particulièrement limitée à ce nombre.

De plus, dans ce premier mode de l'invention, une source (9) de lumière ultraviolette, qui est un laser ultraviolet émettant à une longueur d'onde de 365 nm, éventuellement pulsé pour bénéficier d'une puissance optique importante, est incluse dans le dispositif et éclaire une matrice (10) plane de micro-miroirs commandée digitalement par un ordinateur non représenté. Cette matrice permet de réaliser un objet dont chaque pixel peut être commandé individuellement pour former un motif objet, de complexité quelconque ayant un nombre de points indépendants égal au nombre de micro-miroirs de la matrice. Chaque micro-miroir peut être ramené à un pixel noir ou éclairé, la matrice formant un motif dans son plan, c'est-à-dire un motif à deux dimensions. Cette matrice est imagée par un objectif (11) optimisé pour l'ultraviolet et formant une image de la matrice sur la première surface du substrat, dans le milieu du substrat. Le système optique ou objectif pourra être un microscope inversé, c'est-à-dire un objectif de microscope dans lequel le sens de la lumière est inversé, pour transformer la matrice structurée selon un motif de résolution de l'ordre de la dizaine de microns en une image micro-structurée avec une résolution ou finesse de l'ordre du micron par un grandissement inférieur à 1 de l'ordre de 1/10.

Le substrat est avantageusement disposé de telle façon que ce substrat est d'abord traversé par la lumière émise par le laser avant que l'image ne se forme sur sa première surface. Autrement dit, si le substrat est d'une première épaisseur s'étendant entre la seconde surface et la première surface, la seconde surface est touchée par la lumière ultraviolette puis la première surface est touchée par la lumière ultraviolette, dans le sens de propagation de la lumière. Cette disposition est désignée dans la demande par l'expression « le substrat est éclairé de l'intérieur », par opposition à la situation où la lumière rencontre la première et la seconde surface dans cet ordre qui est désignée par l'expression « le substrat est éclairé de l'extérieur ». L'éclairage par l'intérieur du substrat permet de s'affranchir des défauts optiques de la solution, c'est une variante de réalisation privilégiée pour l'invention.

Les éléments substrat, lumière, benzophénone PEG et protéine sont choisis comme suit : pour une protéine donnée, le PEG est choisi en tant que couche non-adhésive pour la protéine mais adhésive sur le substrat, la benzophénone est choisi en tant que colle adhésive pour la protéine et la couche, sous illumination par la lumière. Il est ainsi possible de déterminer d'autres matériaux permettant la mise en oeuvre de l'invention pour d'autres protéines.

Un dispositif de photo-impression, selon un premier motif structuré, de la première protéine sur le substrat traité est alors obtenu par un procédé comprenant les étapes suivantes :
- remplir le circuit micro-fluidique avec la première solution aqueuse contenant la première protéine jusqu'à mettre en contact la première solution et la couche de PEG, au niveau de l'ouverture du micro-canal recouverte par la couche de PEG déposée sur le substrat.
- former, à l'aide de ladite lumière, une première image micro-structurée, réduite par l'objectif de microscope, d'un premier motif structuré, sur la couche, pour photo-imprimer la première protéine sur la couche.

L'invention occupe ainsi une première configuration, en relation avec une impression au moyen du premier réservoir. Un motif complexe d'impression de deux protéines sur le substrat peut être facilement effectué à partir de cette première configuration, en remplaçant la première solution par la seconde solution et en remplaçant le premier motif affiché ou réfléchi ou propagé ou transmis par la matrice, par un second motif.

Ainsi, il suffit pour obtenir la première configuration d'ouvrir la première micro-vanne et fermer la seconde micro-vanne et de pomper avec la micro-pompe pour obtenir la première protéine au niveau de la couche. Il suffit aussi pour obtenir le premier motif micro-structuré sur la couche, d'alimenter la source de lumière et de sélectionner un premier motif sur la matrice.

Considérant que, dans ce premier mode, la matrice de micro-miroirs possède des moyens optiques de remplacement du premier motif structuré par un second motif structuré différent.

Considérant aussi que, dans ce premier mode, le circuit micro-fluidique, défini comme moyen d'alimentation en solution de la zone constituée par la couche antiadhésive pour les protéines, possède des moyens micro-fluidiques de remplacement de la première solution par une seconde solution, au niveau de la couche.

Il est alors simple de remplacer la première solution aqueuse par la seconde solution aqueuse au niveau de la couche et de remplacer le premier motif structuré par un second motif structuré sur la matrice, second motif dont la seconde image micro-structurée se forme alors sur la couche.

Il en résulte alors une seconde configuration de l'invention dans ce premier mode, dans laquelle la seconde protéine est photo-imprimée sur la couche selon l'image du second motif.

Il est aussi possible de remplacer la première solution par une pluralité de solutions aqueuses de protéines et de molécules photo-activables (sulfo-SANPAH ou Sulfosuccinimidyl 6-((4-azido-2-nitrophenyl)amino)hexanoate, Antraquinone-2-sulfonic acid sodium salt monohydrate, benzophenone-4-maleimide, benzophenone-4-isocyanate, 4-azido-2,3,5,6-tetrafluorobenzoic acid, succinimidyl ester) et de remplacer le premier motif structuré de la matrice de micro-miroirs par une pluralité de motifs, associée à une impression souhaitée pour une protéine d'une solution de la pluralité, pour en obtenir une image micro-structurée sur la couche de PEG. L'invention est ainsi adaptée à l'impression aisée d'une pluralité de protéines sur un substrat selon des motifs arbitrairement choisis donc une impression arbitrairement complexe.

Il est aussi possible d'ajouter une troisième voie de rinçage en ajoutant un troisième réservoir rempli d'une solution tampon, en le reliant à une troisième micro-vanne à commande automatique, de même type que la première et la seconde micro-vanne et en reliant la sortie de cette troisième vanne à la micro-pompe. La solution tampon de rinçage pourra être notamment de l'eau distillée ou du sérum physiologique ou d'autres solution de lavage de protéines (surfactants ioniques ou non-ioniques : Tween 20, Triton X100).

L'invention possède ainsi un intérêt particulier pour une utilisation pour l'impression rapide et versatile de plusieurs protéines sur un substrat, à partir de plusieurs réservoirs et avec un contraste chimique élevé et durable, c'est-à-dire sans greffage non-spécifique. Le contraste chimique est commodément défini, pour la présente demande, comme la différence de concentration d'une même protéine en des lieux distincts, différence divisée par la somme des concentrations de ladite même protéine, en ces mêmes lieux.

L'invention possède aussi une capacité à fonctionner industriellement car il est aussi à noter que dans toutes les configurations de l'invention, un calculateur peut avantageusement piloter, en les dotant de commandes automatiques connues de l'art antérieur, chacun des éléments de l'invention : source de lumière, matrice de micro-miroirs, circuit fluidique (micro-pompe, micro-vannes).

L'utilisation pour l'impression de deux protéines de l'invention est, par exemple, obtenue selon deux configurations.

Dans une première configuration d'utilisation, le premier réservoir (1) est connecté par la première micro-vanne (3) ouverte, à la micro-pompe (5) et au micro-canal (6), la seconde micro-vanne(4) est fermée pour éviter le mélange, dans le circuit micro-fluidique, des fluides contenus dans les premier et second réservoirs. Un premier fluide contenant une première protéine s'écoule ainsi du premier réservoir au micro-canal puis s'échappe dans le réservoir de fuite (8). Dans cette première configuration, un premier motif est dessiné sur la matrice de micro-miroirs (10) par un calculateur non représenté et l'image de ce premier motif ou premier motif optique est formée sur la première surface du substrat. Le premier réservoir contient un premier fluide contenant une première protéine et un premier moyen de greffage en solution, de la première protéine sur la première surface, sous illumination ultraviolette. L'image du premier motif optique, est traduite sous forme d'un premier motif chimique de la première protéine par le premier moyen de greffage, de même étendue spatiale que le premier motif optique et qui lui est superposable.

Dans une seconde configuration d'utilisation, le second réservoir (2) est connecté par la seconde micro-vanne (4) ouverte, à la micro-pompe (5) et au micro-canal (6), la première micro-vanne(3) est fermée pour éviter le mélange des fluides contenus dans les premier et second réservoirs. Un second fluide contenant une seconde protéine s'écoule ainsi du second réservoir au micro-canal puis s'échappe dans le réservoir de fuite (8). Dans cette seconde configuration, un second motif est dessiné sur la matrice de micro-miroirs (10) par le calculateur et l'image de ce second motif ou second motif optique est formée sur la première surface du substrat. Le second réservoir contient un second fluide contenant une seconde protéine et un second moyen de greffage en solution, de la seconde protéine sur la première surface, sous illumination ultraviolette. L'image du second motif optique, est traduite sous forme d'un second motif chimique de la seconde protéine par le second moyen de greffage, de même étendue spatiale que le second motif optique et qui lui est superposable parfaitement, le substrat n'ayant pas bougé dans toute l'utilisation, ce qui constitue un avantage considérable pour une impression d'une pluralité de protéines, pour laquelle les opérations effectuées pour la seconde protéine peuvent être reprise pour chaque protéine restant à imprimer, sans difficulté.

Dans une autre utilisation, l'invention peut servir à la réalisation de gradient de concentration d'une même protéine, notamment en modulant le temps d'illumination du traitement de surface du substrat ou en utilisant un même temps mais en sélectionnant des réservoirs contenant une même protéine à des concentrations différentes.

Il a été notamment obtenu avec l'invention, des gradients de Fibrinogène vert (Alexa 488), de Fibrinogène rouge (Alexa 546) et de Fibrinogène jaune (Alexa 532), en projetant l'image d'une grille à des temps d'illumination différents.

Concernant l'invention en général et pour d'autres modes ou utilisations, les considérations suivantes s'appliquent :
- Toute matrice autre que de micro-miroirs qui propage la lumière selon un motif contrasté optiquement est adaptée à l'invention. Un modulateur spatial à cristaux liquides fonctionnant par transmission plutôt que par réflexion ou par absorption de la lumière serait adapté pour l'invention.
- Tout moyen de créer un motif et d'en faire l'image sur la couche, est adapté à l'invention. Un système par transmission de la lumière de la source, créant un motif contrasté ou motif lumineux ou motif, par transmission de la lumière, le motif étant repris par l'objectif et imagé sur la première surface du substrat par l'objectif serait aussi adapté à l'invention. Le fonctionnement par réflexion de la matrice de micro-miroirs associée à un objectif de microscope inversé, du premier mode de réalisation de l'invention est donc un exemple de moyen d'illumination ou de modulateur spatial de la lumière de la source, au sens de l'invention.

L'invention possède dans tous ses modes, des avantages pratiques pour l'impression de plusieurs protéines :
- Stabilité optique et dimensionnelle du système, aucun démontage n'étant nécessaire, tout en permettant de limiter le greffage non-spécifique des protéines, indispensable dans l'application, par l'utilisation du PEG comme couche d'impression et de la benzophénone en solution.
- Adaptation à un nombre arbitraire de réservoirs et à une pluralité de protéines en augmentant simplement le nombre de micro-vannes et en utilisant une logique d'ouverture de ces micro-vannes dans laquelle une seule micro-vanne est ouverte à la fois, les autres étant fermées. Il est donc ainsi possible d'imprimer un nombre arbitraire de protéines sur un substrat avec l'invention, sans réduction de contraste par rapport à l'impression d'une seule protéine et sans perte de temps, sous réserve de pouvoir disposer d'un moyen de greffage de chaque protéine en solution avec chaque protéine, ce moyen de greffage étant adapté au substrat. Le pilotage de la logique pourra être assuré notamment par un calculateur, pour automatiser au mieux, un système d'impression selon l'invention.
- Fonctionnement éventuel sans voie de rinçage. En effet, Il apparaît de la description du premier mode de l'invention que l'écoulement d'une solution sur la couche contribue au lavage de la première surface lorsqu'il est actif sans illumination. Ce lavage s'applique au lavage du premier fluide par le second fluide lors d'un basculement de la première à la seconde configuration du premier mode. Durant le basculement, c'est-à-dire le changement de fluide en contact avec la première surface, ou phase de basculement, un mélange de fluide est au contact de la première surface, il peut alors être souhaitable de couper l'illumination pour éviter une fixation ou impression d'un mélange de protéines. L'illumination peut être rétablie lorsqu'il est estimé qu'il n'y a en contact avec la première face, qu'un fluide pur à la précision recherchée pour l'impression, c'est à dire lorsqu'un contraste chimique recherché pour une seule protéine sur la première surface peut être atteint. Une illumination intermittente peut être facilement déterminée par un homme du métier sur le critère du contraste obtenu pour chaque protéine imprimée.

L'invention est susceptible d'application industrielle dans le domaine de l'impression de protéines sur un substrat.

Les considérations ci-dessous s'appliquent aussi à l'invention.

Il est entendu au sens de la présente demande ou ici, que ledit système micro-fluidique pourra comprendre un circuit permettant le maintien du substrat sous vide. Ce qui offre la possibilité de récupérer le substrat traité aisément par rupture du vide, en fin d'impression.

Il est entendu ici, qu'il est conforme à l'enseignement de l'invention, pour tous ses dispositifs, que ledit circuit micro-fluidique comprenne un circuit de lavage relié à une solution tampon comme un sérum physiologique ou une autre solution compatible avec les protéines à imprimer, et des moyens de lavage de ladite couche vis-à-vis des solutions des protéines à imprimer. Dans ce cas, la couche d'impression peut-être lavée in-situ, ce qui permet de ne pas déplacer le substrat par rapport aux images des motifs à imprimer, lors d'un remplacement d'une solution de protéine à y imprimer et permet de maintenir une grande précision d'impression relative entre des motifs de protéines différentes ou différents motifs d'une même protéine.

Il est entendu ici que dans les utilisations de dispositifs selon l'invention comprenant un circuit de lavage relié à une solution tampon et lesdits moyens de lavage, une étape consistant à remplacer ladite première solution aqueuse par ladite seconde solution aqueuse pour mettre en contact la seconde solution et ladite couche, au niveau de ladite ouverture pourra comprendre les sous-étapes suivantes :
- remplacer ladite première solution aqueuse par ladite solution tampon, par les moyens de lavage, et
- remplacer la solution tampon par la seconde solution aqueuse pour mettre en contact la seconde solution et ladite couche, au niveau de ladite ouverture.

Il est entendu ici que dans un dispositif selon l'invention comprenant ledit circuit de lavage relié à une solution tampon et lesdits moyens de lavage, et des moyens d'imprimer un ensemble de N motifs en bijection avec un ensemble de N protéines, un exemple pratique de procédé d'utilisation du dispositif pourra comprendre l'étape suivante :
- pour chaque protéine i, i variant de 1 à N, à effectuer les sous-étapes suivantes :
   ∘ ouvrir une micro-vanne i contrôlant un réservoir i contenant une solution aqueuse photo-activable, au sens d'une solution aqueuse contenant au moins une molécule photo-activable, la solution aqueuse contenant aussi une protéine i pour permettre de mettre en contact ladite couche avec la solution aqueuse de la protéine i,
   ∘ imprimer le motif i par éclairage de la couche au moyen d'une source, notamment ultraviolette (UV) permettant l'impression de la protéine i sur la couche,
   ∘ supprimer l'éclairage de la source,
   ∘ fermer la micro-vanne i, et
   ∘ ouvrir une micro-vanne tampon contrôlant un réservoir tampon contenant une solution tampon pour laver la couche de la solution aqueuse de la protéine i par les moyens de lavage.

Il est entendu ici que l'invention est aussi un dispositif selon la revendication 1, dans lequel les moyens micro-fluidiques de remplacement de la première solution par la seconde solution comprennent des moyens de lavage de la couche par une solution tampon compatible avec la première et la seconde protéine, lesdits moyens de lavage étant aptes à laver la couche de la solution aqueuse de la première protéine.

## Revendications

1. Dispositif pour le greffage micro-structuré de plusieurs protéines sur un substrat, comprenant:
- un substrat (7),
- une couche de polyéthylène glycol disposée sur le substrat,
- une matrice adaptée à propager la lumière selon un premier motif structuré et comprenant des moyens optiques de remplacement du premier motif structuré par un second motif structuré,
- une source d'une lumière (9) adaptée à éclairer la matrice,
- un objectif (11) de microscope adapté à former, sur la couche, une première image micro-structurée du premier motif,
- un premier réservoir (1) contenant une première solution aqueuse comprenant une benzophénone et une première protéine,
- un deuxième réservoir (2) destiné à recevoir une seconde solution aqueuse comprenant une benzophénone et une seconde protéine, et
- un circuit micro-fluidique adapté à contenir la première solution aqueuse, comprenant une ouverture adaptée pour mettre en contact la première solution avec la couche au niveau de l'ouverture,
dans lequel:
- le circuit micro-fluidique est adapté pour être rempli avec la première solution aqueuse pour mettre en contact la première solution aqueuse et ladite couche, la première image micro-structurée du premier motif étant formée sur la couche au moyen de la source de lumière pour photo-imprimer la première protéine sur la couche,
- le circuit micro-fluidique comprend des moyens micro-fluidiques de remplacement de la première solution par la seconde solution, pour mettre en contact la deuxième solution aqueuse et ladite couche, le premier motif étant remplacé par le deuxième motif pour photo-imprimer la deuxième protéine sur la couche, et
- la matrice est une matrice (10) plane de micro-miroirs propageant la lumière par réflexion.

2. Dispositif selon la revendication 1, dans lequel ladite source de lumière est un laser émettant à une longueur d'onde ultraviolette.

3. Dispositif selon la revendication 2, dans lequel ladite longueur d'onde ultraviolette vaut 365 nm.

4. Procédé de greffage micro-structuré de protéines sur un substrat utilisant un dispositif selon l'une quelconque des revendications 1 à 3 et comprenant les étapes suivantes :
- remplir le premier réservoir avec une première solution aqueuse comprenant une benzophénone et une première protéine,
- remplir ledit circuit micro-fluidique avec ladite première solution aqueuse pour mettre en contact la première solution et ladite couche, au niveau de ladite ouverture, et
- former, à l'aide de ladite lumière, ladite première image micro-structurée dudit premier motif structuré, sur la couche, pour photo-imprimer ladite première protéine sur la couche.

5. Procédé selon la revendication 4, dans lequel ladite première protéine est fluorescente.

6. Procédé selon l'une quelconque des revendications 4 à 5 comprenant les étapes suivantes :
- remplir le deuxième réservoir avec une deuxième solution aqueuse comprenant la benzophénone et une deuxième protéine,
- remplacer ladite première solution aqueuse par ladite seconde solution aqueuse pour mettre en contact la seconde solution et ladite couche, au niveau de ladite ouverture, et
- remplacer ledit premier motif structuré par ledit second motif structuré pour photo-imprimer ladite seconde protéine sur la couche.

7. Procédé selon l'une quelconque des revendications 4 à 6, dans lequel ladite seconde protéine est fluorescente.

## Patentansprüche

1. Vorrichtung zur mikrostrukturierten Pfropfung von mehreren Proteinen auf ein Substrat, umfassend:
- ein Substrat (7),
- eine auf dem Substrat angeordnete Schicht aus Polyethylenglycol,
- eine Matrix, welche geeignet ist, Licht gemäß einem ersten strukturierten Muster weiterzuleiten, und welche optische Mittel zum Ersetzen des ersten strukturierten Musters durch ein zweites strukturiertes Muster umfasst,
- eine Lichtquelle (9), welche geeignet ist, die Matrix zu beleuchten,
- ein Mikroskopobjektiv (11), welches geeignet ist, auf der Schicht ein erstes mikro strukturiertes Bild des ersten Musters zu bilden,
- ein erstes Reservoir (1), welches eine erste wässrige Lösung enthält, welche ein Benzophenon und ein erstes Protein umfasst,
- ein zweites Reservoir (2), welches dazu konfiguriert ist, eine zweite wässrige Lösung aufzunehmen, welche ein Benzophenon und ein zweites Protein umfasst, und
- einen Mikrofluidkreislauf, welcher geeignet ist, die erste wässrige Lösung zu enthalten, welcher eine Öffnung umfasst, die dazu konfiguriert ist, die erste Lösung mit der Schicht an der Öffnung in Kontakt zu bringen,
wobei:
- der Mikrofluidkreislauf dazu konfiguriert ist, mit der ersten wässrigen Lösung befüllt zu werden, um die erste wässrige Lösung mit der Schicht in Kontakt zu bringen, das erste mikro strukturierte Bild des ersten Musters auf der Schicht mittels der Lichtquelle gebildet wird, um das erste Protein auf der Schicht fotographisch zu drucken,
- der Mikrofluidkreislauf mikrofluidische Mittel zum Ersetzen der ersten Lösung durch die zweite Lösung umfasst, um die zweite wässrige Lösung und die Schicht in Kontakt zu bringen, das erste Muster durch das zweite Muster ersetzt wird, um das zweite Protein auf der Schicht fotographisch zu drucken, und
- die Matrix eine ebene Matrix (10) aus Mikrospiegeln ist, welche das Licht durch Reflektion weiterleiten.

2. Vorrichtung nach Anspruch 1, wobei die Lichtquelle ein Laser ist, welcher bei einer ultravioletten Wellenlänge emittiert.

3. Vorrichtung nach Anspruch 2, wobei die ultraviolette Wellenlänge 365 nm ist.

4. Prozess der mikrostrukturierten Pfropfung von Proteinen auf ein Substrat unter Verwendung einer Vorrichtung nach einem der Ansprüche 1 bis 3 und die folgenden Schritte umfassend:
- Befüllen des erstes Reservoirs mit einer ersten wässrigen Lösung, welche ein Benzophenon und ein erstes Protein umfasst,
- Befüllen des Mikrofluidkreislaufs mit der ersten wässrigen Lösung, um die erste Lösung an der Öffnung mit der Schicht in Kontakt zu bringen, und
- Bilden, mit der Hilfe des Lichts, des ersten mikrostrukturierten Bildes des ersten strukturierten Musters auf der Schicht, um das erste Protein auf der Schicht fotographisch zu drucken.

5. Prozess nach Anspruch 4, wobei das erste Protein fluoreszierend ist.

6. Prozess nach einem der Ansprüche 4 bis 5, die folgenden Schritte umfassend:
- Befüllen des zweiten Reservoirs mit einer zweiten wässrigen Lösung, welche das Benzophenon und ein zweites Protein umfasst,
- Ersetzen der ersten wässrigen Lösung durch die zweite wässrige Lösung, um die zweite Lösung und die Schicht an der Öffnung in Kontakt zu bringen, und
- Ersetzen des ersten strukturierten Musters durch das zweite strukturierte Muster, um das zweite Protein auf der Schicht fotographisch zu drucken.

7. Prozess nach einem der Ansprüche 4 bis 6, wobei das zweite Protein fluoreszierend ist.

## Claims

1. A device for the microstructured grafting of several proteins onto a substrate, comprising:
a substrate (7),
a layer of polyethylene glycol placed on the substrate,
a matrix for propagating the light in a first structured pattern and comprising optical means for replacing the first structured pattern with a second structured pattern,
a light source (9) adapted for illuminating the matrix,
microscope objective (11) for forming, on the layer, a first microstructured image of the first pattern,
a first container (1) for receiving a first aqueous solution comprising a benzophenone and a first protein,
a second container (2) for receiving a second aqueous solution comprising benzophenone and a second protein, and
a microfluidic circuit adapted for containing the first aqueous solution, comprising an opening for bringing the first solution into contact with the layer at the opening,
wherein:
the microfluidic circuit is adapted to be filled with the first aqueous solution so as to bring the first aqueous solution and the layer into contact, the first microstructured image of the first structured pattern being formed on the layer by means of the light source in order to photoprint the first protein on the layer,
the microfluidic circuit comprises microfluidic means for replacing the first aqueous solution with the second aqueous solution so as to bring the second aqueous solution and the layer into contact, the first structured pattern being replaced by the second pattern in order to photoprint the second protein on the layer, and
the matrix is a plane matrix (10) of micromirrors propagating the light by reflection.

2. The device as claimed in claim 1, wherein said light source is a laser emitting at an ultraviolet wavelength.

3. The device as claimed in claim 2, wherein said ultraviolet wavelength is 365 nm.

4. A method for the microstructured grafting of proteins onto a substrate using a device as claimed in any one of claims 1 to 3 and comprising the following steps:
- filling the first container with a first aqueous solution comprising a benzophenone and a first protein,
- filling said microfluidic circuit with said first aqueous solution so as to bring the first solution and said layer into contact, at said opening, and
- forming, by means of said light, said first microstructured image of said first structured pattern, on the layer, in order to photoprint said first protein on the layer.

5. The method as claimed in claim 4, wherein said first protein is fluorescent.

6. The method as claimed in any one of claims 4 to 5, comprising the following steps:
- filling the second container with a second aqueous solution comprising the benzophenone and a second protein,
- replacing said first aqueous solution with said second aqueous solution so as to bring the second solution and said layer into contact, at said opening, and
- replacing said first structured pattern with said second structured pattern in order to photoprint said second protein on the layer.

7. The method as claimed in any one of claims 4 to 6, wherein said second protein is fluorescent.
